# DEMANDE DE BREVET EUROPEEN

(11) **EP 0 962 453 A1**
(43) Date de publication de la demande: **08.12.1999**
(21) Numéro de dépôt: 99401132.8
(22) Date de dépôt: 07.05.1999
(51) Int. Cl.: C07D 233/64, A61K 7/48, C07K 5/06

(54) **Nouveaux dérivés d'histidine, procédé de préparation et utilisations**

(30) Priorité: 25.05.1998 FR 9806538
(71) Demandeur: L'OREAL, 75008 Paris (FR)
(72) Inventeur: Philippe, Michel, 91320 Wissous (FR); Bordier, Thierry, 93290 Tremblay en France (FR)
(74) Mandataire: Renard, Emmanuelle

(57) **Abrégé**

L'invention concerne de nouveaux dérivés d'histidine répondant à la formule générale (I) suivante : 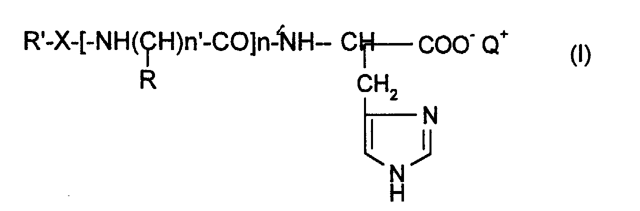 dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite :
   -NH-CO-, -SO2-, -NH-CO-CO-, -O-CO-CO-,
R' représente un radical alkyle linéaire ou ramifié, saturé ou insaturé, éventuellement hydroxylé, en C6 à C22 ou un radical aminoalkyle en C6 à C22, la fonction amine étant événtuellement protégée sous forme d'acétamide ou substitué par un ou deux groupements alkyles inférieurs,
Q⁺ représente H⁺ ou un cation organique ou minéral,
ainsi que les sels d'addition d'un composé de formule (I) avec un acide organique ou minéral. L'invention concerne aussi bien les composés à configuration D, à configuration L que leurs mélanges, en particulier le mélange racémique des composés D et L.

L'invention concerne également un procédé de préparation des composés de formule (I) ainsi que l'utilisation de ces composés en cosmétique et en pharmacie.

## Description

La présente invention a pour objet de nouveaux dérivés d'histidine et leur procédé de préparation. Elle a également pour objet des compositions cosmétiques ou dermatologiques comprenant ces composés. Elle a encore plus particulièrement pour objet l'utilisation de ces composés comme agents anti-radicaux libres.

Le rayonnement solaire, la chaleur, la pollution atmosphérique et notamment les fumées et le tabac sont connus pour entraîner la formation des radicaux libres. Ils proviennent en grande partie de l'oxygène moléculaire.

On peut citer les radicaux libres suivants:
- l'oxygène singulet, très oxydant, très toxique et d'une durée de vie très courte, produit de l'excitation de l'oxygène moléculaire par les photons de la lumière;
- le radical anion superoxyde, produit de l'addition d'un électron sur l'oxygène et pouvant donner lieu à la production des radicaux hydroxyles très réactifs;
- le radical hydroxyle, très oxydant et le plus toxique pour les cellules.

La formation de ces espèces radicalaires conduisent notamment à l'oxydation des lipides cutanés.

Les cellules vivantes, notamment celles de la peau, du cuir chevelu et de certaines muqueuses, sont particulièrement sensibles à ces radicaux libres, ce qui se traduit par un vieillissement accéléré de la peau, avec un teint manquant d'éclat et une formation précoce de rides ou de ridules, et aussi par une diminution de la vigueur et un aspect terne des cheveux.

Il est donc paru particulièrement important de protéger la peau, les cheveux et les muqueuses de ces radicaux libres.

Il est connu que certains anti-oxydants sont susceptibles d'inhiber la formation de radicaux libres.

Ainsi, la carnosine, ou N-β-alanyl-L-histidine, qui est un dipeptide naturel se trouvant dans les muscles de nombreux vertébrés, est connue pour son activité anti-radicaux libres, en particulier anti oxygène singulet. (E. Decker et H. Faraji, JAOCS, vol 67, n° 10, 650-652, 1990). Son utilisation comme agent-antioxydant ou comme agent anti-radicaux libre en cosmétique est aussi connue par la demande WO-A-92/09298. Cependant, la carnosine présente au contact de la peau des problèmes de dégradation causés par les enzymes présentes dans la peau et notamment les protéases, ce qui conduit à une perte sensible de son activité.

Des dérivés d'histidine sont aussi connus, comme par exemple les dérivés N-(4-amino-1-oxobutyl)-L-histidine, N-(5-amino-1-oxopentyl)-L-histidine, N-(6-amino-1-oxohexyl)-L-histidine décrits dans RU2084457. Toutefois, du fait de leur nature physico-chimique, de tels dérivés d'histidine ne sont pas satisfaisants pour protéger de l'oxydation des composés apolaires.

La demanderesse a découvert de façon inattendue de nouveaux dérivés d'histidine présentant une meilleure activité anti-radicaux libre vis-à-vis des composés apolaires oxydables et notamment ayant une bonne propriété d'efficacité dans la désactivation de l'oxygène singulet. Ils peuvent donc être utilisés en cosmétique et en pharmacie : ils sont facilement applicables sur la peau.

La demanderesse a constaté que de tels résultats pouvaient être obtenus avec des dérivés de l'histidine.

L'invention a donc pour objet de nouveaux dérivés d'histidine répondant à la formule générale (I) suivante : 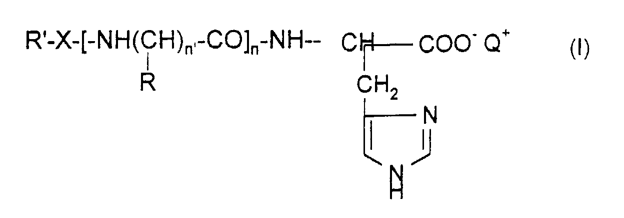 dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite :

   -NH-CO-, -SO₂-, -NH-CO-CO-, -O-CO-CO-,
R' représente un radical alkyle linéaire ou ramifié, saturé ou insaturé, éventuellement hydroxylé, de C₆ à C₂₂ ou un radical aminoalkyle de C6 à C22, la fonction amine étant événtuellement protégée sous forme d'acétamide ou substitué par un ou deux groupements alkyles inférieurs,
Q⁺ représente H⁺ ou un cation organique ou minéral,
ainsi que les sels d'addition d'un composé de formule (I) avec un acide organique ou minéral.

Le cation organique (Q⁺) peut être choisi parmi les ammoniums comportant un reste choisi parmi les aminoacides basiques tels que la lysine, l'arginine, ou bien encore parmi les amino-alcools tels que la glucamine, la N-méthyl glucamine, l'amino-3 propanediol-1,2.

Le cation minéral (Q⁺) peut être choisi parmi les cations alcalins ou alcalino-terreux tels que Na⁺, K⁺, ou peut être l'ion NH₄⁺.

Les sels d'addition avec un acide sont choisis par exemple parmi les chlorhydrates, les bromhydrates, les sulfates, les tartrates et les acétates.

Les composés de formule (I) comportent dans leur structure chimique au moins un carbone asymétrique : l'invention concerne aussi bien les composés à configuration D, à configuration L que leurs mélanges, en particulier le mélange racémique des composés D et L.

Selon l'invention, les composés préférés de formule (I) présentent au moins une des caractéristiques suivantes :
- R' désigne de préférence un radical alkyle, linéaire ou ramifié, saturé, ayant de 8 à 18 atomes de carbone,
- n est un entier de 1 à 5, et
- n' est un entier allant de 1 à 11.

Selon la présente invention, parmi les radicaux alkyle linéaire ou ramifié ayant de 6 à 22 atomes de carbone, on peut citer avantageusement les radicaux hexyle, octyle, nonyle, 2-éthyl-hexyl, dodécyle, hexadécyle et octadécyle.

Les groupements alkyle inférieurs comprennent généralement de 1 à 6 atomes de carbone. On peut citer, comme groupement alkyle inférieur, les radicaux méthyle, éthyle, propyle, isopropyle, tertiobutyle, hexyle.

Parmi les radicaux alkyle linéaire ayant de 6 à 22 atomes de carbone, on peut citer notamment les radicaux octyle, dodécyle, hexadécyle et octadécyle.

Parmi les radicaux alkyle ramifiés ayant de 6 à 22 atomes de carbone, on peut citer notamment les radicaux 2-méthylpentyle, 1-méthylhexyle, 3-méthylheptyle.

Par radical alkyle insaturé, on préfère un radical ayant de 6 à 22 atomes de carbone linéaire ou ramifié comportant une ou plusieurs doubles liaisons.

Les chaînes latérales d'un acide aminé correspondent aux chaînes latérales de l'un quelconque des amino-acides naturels. Ainsi, R peut représenter notamment l'hydrogène, un radical méthyle ou isopropyle. Cela peut donc être des chaînes latérales non polaires, polaires mais non chargées, chargées négativement ou positivement.

Parmi les composés préférés correspondant à la formule générale (I), on peut notamment citer :
- la N-octylaminocarbonyl-β-alanyl-L-histidine,
- la N-dodecylaminocarbonyl-β-alanyl-L-histidine,
- la N-octylsulfonyl-β-alanyl-L-histidine,
- la N-dodecylsulfonyl-β-alanyl-L-histidine,
- la N-dodecylamino-oxalyl-β-alanyl-L-histidine.

La présente invention a également pour objet le procédé de préparation des composés de formule (I). Ce procédé comprend la réaction avec l'histidine dans un solvant inerte, d'un composé de formule (Il) 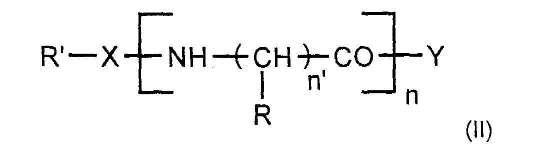 dans laquelle n', n, R, R' et X ont les mêmes significations que dans la formule (I) ci-dessus, Y est un groupement activant classique de la fonction acide.

Les réactions d'activation des groupements acides -COOH sont bien connues de l'homme du métier. On peut par exemple se reporter à "Advanced Organic Chemistry, Jerry March, 3^{ème} édition, 1985, p.370-377". On entend par agent de couplage tout composé susceptible de substituer le groupe OH du composé de formule (IV), puis d'être substitué ensuite par l'acide aminé que l'on souhaite greffer, par exemple l'histidine. Des agents de couplage sont cités dans "Advanced organic chemistry, J March, 3^{ème} édition, 1985, page 372". On peut notamment utiliser comme agent de couplage le 2-(5-norbornène-2,3-dicarboximido)-1,1,3,3 tétraméthyluronium tétrafluoroborate.

L'histidine de départ comportant un carbone asymétrique, est utilisée dans la forme optique, pure ou en mélange (D; L; D,L) selon la forme optique désirée du composé de formule (I).

Comme solvant, on peut utiliser le dichlorométhane, le dichloro-1,2 éthane, le trichloro-1,1,1 éthane, le chloroforme, l'acétonitrile, le toluène, le dioxane, le tétrahydrofurane, le diméthoxy-1,2 éthane, le cyclohexane, le diméthylformamide, l'eau ou un mélange de ces solvants.

La réaction est effectuée à une température comprise de préférence entre -10°C et + 40°C, et plus préférentiellement entre 20°C et 30°C.

La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine, la di-isopropyl éthylamine ou la triéthylamine. On utilise de préférence l'hydrogénocarbonate de sodium.

La présente invention a également pour objet une composition comprenant, dans un milieu physiologiquement acceptable, un composé de formule (I) tel que défini ci-dessus.

La composition comprenant ledit composé peut se présenter notamment sous forme d'une composition cosmétique ou pharmaceutique comprenant respectivement un milieu cosmétiquement ou pharmaceutiquement acceptable.

Dans les compositions selon l'invention, les composés de formule (I) sont en général présents à une concentration de 0,001% à 15% en poids et de préférence de 0,01% à 5% en poids par rapport au poids total de la composition.

Ces compositions peuvent être préparées selon les méthodes usuelles connues de l'homme de l'art. Elles peuvent être sous forme de lotion, de gel, d'émulsion eau-dans-huile ou huile-dans-eau, de microémulsion, de lait ou de crème, de poudre, de pâtes, de stick solide, de spray ou de mousse aérosol.

L'invention a également pour objet l'utilisation des composés de formule (I) comme agent anti-radicaux libres, et notamment comme agent anti-radicaux libres désactivant l'oxygène singulet, et notamment dans une composition cosmétique ou pharmaceutique.

L'invention concerne également l'utilisation des composés de formule (I) dans une composition cosmétique ou pharmaceutique pour le traitement des matières kératiniques contre les effets du vieillissement. Par matières kératiniques, on entend la peau, les cheveux, les ongles, les poils, les muqueuses et les semi-muqueuses telles que les lèvres.

Les composés de formule (I) constituent des lipides amphiphiles anioniques susceptibles d'être inclus dans un système vésiculaire.

Les compositions contenant les composés selon l'invention peuvent également comporter, de façon connue, un ou plusieurs composés actifs ayant une activité cosmétique et/ou pharmaceutique qui, selon leurs caractéristiques de solubilité, peuvent avoir différentes localisations. Par exemple, dans le cas des dispersions de vésicules contenant une phase aqueuse encapsulée, si les actifs sont liposolubles, ils peuvent être présents dans la phase lipidique constituant le(s) feuillet(s) des vésicules ou dans les gouttelettes de liquide non miscible à l'eau stabilisées par les vésicules. Si les actifs sont hydrosolubles, ils peuvent être présents dans la phase aqueuse encapsulée des vésicules ou dans la phase aqueuse continue de la dispersion. Si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée. De façon générale, les actifs sont mis en place dans la phase lipidique des feuillets et/ou dans la phase encapsulée par les feuillets.

Les compositions selon l'invention peuvent également comprendre, de façon connue, des additifs de formulation n'ayant ni activité cosmétique, ni activité pharmaceutique propre, mais utiles pour la formulation des compositions. Parmi ces additifs, on peut citer par exemple les gélifiants, les polymères, les conservateurs, les colorants, les opacifiants et les parfums.

Les compositions cosmétiques ou pharmaceutiques selon l'invention peuvent se présenter notamment sous forme de shampooings ou d'après-shampooings, de compositions nettoyantes, de crèmes pour le soin de la peau ou des cheveux, de compositions anti-solaires, de crèmes ou de mousses après-rasage, de déodorants corporels, de compositions à usage bucco-dentaire, de compositions tinctoriales capillaires, ou encore de composition de maquillage par exemple.

L'invention sera maintenant illustrée à l'aide des exemples non limitatifs suivants :

### Exemple 1: N-octylsulfonyl-β alanyl-L-histidine

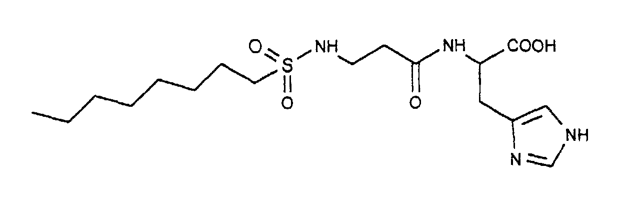

### a) Synthèse de la N-octylsulfonyl-β alanine

Dans un tricol de 1 L muni de deux ampoules à introduction de 250 ml et d'une électrode de verre pour mesurer le pH, 2 g (22,45 mmoles) de β alanine sont dissous dans 1 équivalent de soude 1N. A la température ambiante, 1 équivalent de chlorure de 1-octane sulfonyle dans du tétrahydrofurane est introduit goutte à goutte au mélange réactionnel. Le pH est maintenu supérieur à 9 par l'addition simultanée d'un équivalent de soude 1N. Aprés 3 heures sous agitation à la température ambiante, le mélange est acidifié par environ 1,1 équivalent d'une solution d'acide chlorhydrique 3N. Le mélange hétérogène est extrait par 100 ml d'acétate d'éthyle. La phase organique est lavée par 3 fois 20 ml d'eau, séchée sur sulfate de sodium puis évaporée à sec. Le résidu solide est repris par de l'heptane puis est filtré et séché sous vide. On obtient 1,6g d'un produit blanc, soit un rendement de 30%.

### Analyses:

- Point de fusion : 121,8° C (Mettler FP89)
- Analyse élémentaire (C₁₁H₂₃NO₄S, PM= 265,372)

| | C | H | N | O | S |
|---|---|---|---|---|---|
| % calculé | 49.79 | 8.74 | 5.28 | 24.12 | 12.08 |
| % trouvé | 49.72 | 8.76 | 5.24 | 24.18 | 12.10 |

### b) Synthèse de la N-octylsulfonyl-β-alanyl-L-histidine

Dans un tricol de 100ml muni d'un thermomètre et d'une ampoule à introduction de 10 ml, 4 g (15,07 mmoles) de N-octylsulfonyl-β alanine sont dissous dans 50 ml de tétrahydrofurane et 1 équivalent de triéthylamine. Puis est introduit 1 équivalent de chlorure de pivaloyle à la température de 5°C. Le mélange est ensuite agité pendant 1 heure à la température de 20°C. Une solution contenant 1,2 équivalent d'histidine et 1,2 équivalent de soude dans 20 ml d'eau est préparée puis coulée goutte à goutte dans le mélange réactionnel en maintenant la température inférieure à 30°C. Après 6 heures sous agitation, le mélange est neutralisé par 1 équivalent d'acide chlorhydrique 5N. Le solvant est évaporé puis 50 ml d'eau sont introduits. Le précipité est filtré, lavé à l'eau puis séché sous vide sur diphosphore pentaoxyde. Le produit brut est lavé à l'acétone puis recristallisé dans l'éthanol 95. On obtient 1,6g d'un produit blanc, soit un rendement de 27%.

### Analyses:

- Point de fusion : 146.8° C (Mettler FP89)
- Chromatographie sur couche mince (SiO₂), éluant NH₄OH 6/ CH₃OH 47/ CH₂Cl₂ 47 : Rf= 0.74 (révélation iode)

### Exemple 2: N-octylaminocarbonyl-β alanyl-L-histidine

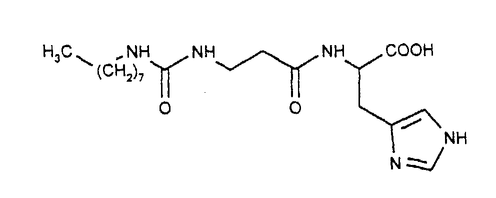

### a) Synthèse de la N-octylaminocarbonyl-β alanine

Dans un tricol de 100 ml, sous atmosphère d'azote, 4g (24,67 mmoles) de 1,1'-carbonyl-diimidazol sont dissous dans 40 ml de diméthylformamide. Puis 4g (24,67 mmoles) d'octylamine sont introduits. Aprés 45 minutes sous agitation à la température ambiante, une solution de 2,4g (1,1 équivalent) de β-alanine dans 10 ml de soude aqueuse (1,1 équivalent), est ajoutée goutte à goutte au mélange précédent. Le mélange devient hétérogène, lequel est ensuite neutralisé par une solution d'acide chlorhydrique concentré dilué au demi. Le précipité est filtré, lavé à l'eau puis séché sous vide sur du diphosphore pentaoxyde. On obtient 4,9g d'un produit blanc, soit un rendement de 82%.

### Analyses:

- Point de fusion : 136.9° C (Mettler FP89)
- Analyse élémentaire (C₁₂H₂₄N₂O₃, PM= 244,333)

| | C | H | N | O |
|---|---|---|---|---|
| % calculé | 58.99 | 9.9 | 11.47 | 19.64 |
| % trouvé | 58.82 | 9.96 | 11.12 | 19.61 |

### b) Synthèse de la N-octylaminocarbonyl-β alanyl-L-histidine

Dans un tricol de 100ml muni d'un thermomètre et d'une ampoule à introduction de 10 ml, 3 g (12,28 mmoles) de N-octylaminocarbonyl-β alanine sont dissous dans 60 ml de tétrahydrofurane et 1 équivalent de triéthylamine. Puis est introduit 1 équivalent de chlorure de pivaloyle à la température de 5°C. Le mélange est ensuite agité pendant 1 heure à la température de 20°C. Une solution contenant 1,2 équivalent d'histidine et 1,2 équivalent de soude dans 20 ml d'eau est préparée puis coulée goutte à goutte dans le mélange réactionnel en maintenant la température inférieure à 30°C. Aprés 6 heures sous agitation, le mélange est neutralisé par 1 équivalent d'acide chlorhydrique 5N. Le solvant est évaporé puis 50 ml d'eau sont introduits. Le précipité est filtré, lavé à l'eau puis séché sous vide sur diphosphore pentaoxyde. Le produit brut est lavé à l'acétone puis recristallisé dans l'éthanol 95. On obtient 1,4g d'un produit blanc, soit un rendement de 30%.

### Analyses:

- Point de fusion : 199.6° C (Mettler FP89)
- Chromatographie sur couche mince (SiO₂):,éluant NH₄OH 6/ CH₃OH 47/ CH₂Cl₂ 47 : Rf= 0.82 (révélation iode).

## Revendications

1. Dérivé d'histidine de formule générale (I) suivante : 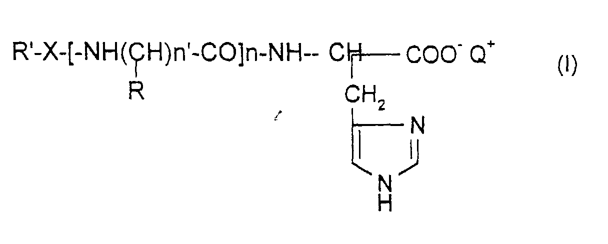 dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite :
-NH-CO-, -SO2-, -NH-CO-CO-, -O-CO-CO-,
R' représente un radical alkyle linéaire ou ramifié, saturé ou insaturé, éventuellement hydroxylé, de C6 à C22 ou un radical aminoalkyle de C6 à C22, la fonction amine étant événtuellement protégée sous forme d'acétamide ou substitué par un ou deux groupements alkyles inférieurs,
Q⁺ représente H⁺ ou un cation organique ou minéral,
ainsi que les sels d'addition d'un composé de formule (I) avec un acide organique ou minéral.

2. Dérivé d'histidine selon la revendication précédente, dans lequel le cation organique est choisi parmi les ammoniums comportant un reste choisi parmi les aminoacides basiques ou les amino-alcools.

3. Dérivé d'histidine selon la revendication 1, dans lequel le cation minéral est choisi parmi le groupe formé par les sels alcalins, les sels alcalino-terreux, l'ion NH₄⁺.

4. Dérivé d'histidine selon la revendication 1, dans lequel les sels d'addition avec un acide sont choisis parmi les chlorhydrates, les bromhydrates, les sulfates, les tartrates et les acétates.

5. Dérivé d'histidine selon l'une quelconque des revendications précédentes, caractérisés en ce qu'il présente au moins une des caractéristiques suivantes :
• R' désigne de préférence un radical alkyle, linéaire ou ramifié, saturé, ayant de 8 à 18 atomes de carbone,
• n est un entier de 1 à 5, et
• n' est un entier allant de 1 à 11.

6. Dérivé d'histidine selon l'une quelconque des revendications précédentes, caractérisés en ce qu'il est choisi parmi la N-octylaminocarbonyl-β-alanyl-L-histidine, la N-dodecylaminocarbonyl-β-alanyl-L-histidine, la N-octylsulfonyl-β-alanyl-L-histidine, la N-dodecylsulfonyl-β-alanyl-L-histidine et la N-dodecylamino-oxalyl-β-alanyl-L-histidine.

7. Procédé de préparation des composés de formule générale (I) suivante : 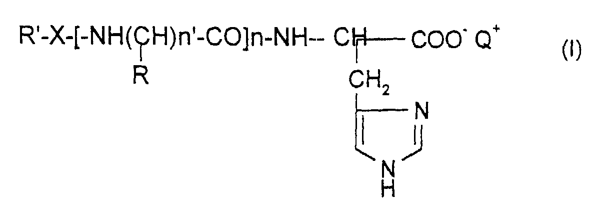 dans laquelle :
n est un entier allant de 0 à 5,
n' est un entier allant de 1 à 16,
R représente une chaîne latérale d'un acide aminé,
X représente un radical choisi parmi les radicaux de formules, lues de gauche à droite :
-NH-CO-, -SO2-, -NH-CO-CO-, -O-CO-CO-,
R' représente un radical alkyle linéaire ou ramifié, saturé ou insaturé, éventuellement hydroxylé, de C6 à C22 ou un radical aminoalkyle de C6 à C22, la fonction amine étant événtuellement protégée sous forme d'acétamide ou substitué par un ou deux groupements alkyles inférieurs,
Q⁺ représente H⁺ ou un cation organique ou minéral,
ainsi que les sels d'addition d'un composé de formule (I) avec un acide organique ou minéral, ce procédé étant caractérisé par le fait qu'il comprend la réaction avec l'histidine dans un solvant inerte, d'un composé de formule (II) 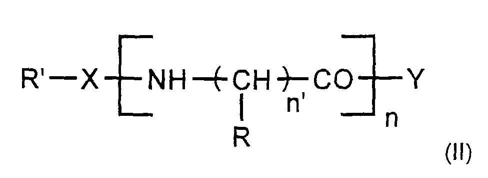 dans laquelle n', n, R, R' et X ont les mêmes significations que dans la formule (I) ci-dessus, Y est un groupement activant la fonction acide.

8. Composition caractérisée par le fait qu'elle comprend, dans un milieu physiologiquement acceptable, au moins un composé de formule (I) tel que défini dans l'une des revendications 1 à 6.

9. Composition selon la revendication 8, caractérisée par le fait qu'elle se présente sous la forme d'une composition cosmétique ou pharmaceutique.

10. Composition selon l'une des revendications 8 ou 9, caractérisée par le fait qu'elle se présente sous la forme de lotion, de gel, d'émulsion, de microémulsion, de lait ou de crème, de poudre, de pâte, de stick solide, de spray ou de mousse aérosol.

11. Composition selon l'une des revendications 8 à 10, caractérisée par le fait que le composé de formule (I) est présent à une concentration de 0,001% à 15% en poids, de préférence 0,005% à 5% en poids, par rapport au poids total de la composition.

12. Utilisation des composés de formule (I) selon l'une des revendications 1 à 6 comme agent anti-radicaux libres.

13. Utilisation des composés de formule (I) selon l'une des revendications 1 à 6 comme agent anti-radicaux libres désactivant l'oxygène singulet.

14. Utilisation des composés de formule (I) selon l'une des revendications 1 à 6 pour la préparation d'une composition cosmétique ou pharmaceutique destinée au traitement des matières kératiniques contre les effets du vieillissement.
